# EUROPÄISCHE PATENTSCHRIFT

(11) **EP 1 952 779 B1**
(45) Veröffentlichungstag und Bekanntmachung des Hinweises auf die Patenterteilung: **04.04.2012**
(21) Anmeldenummer: 07002217.3
(22) Anmeldetag: 01.02.2007
(51) Int. Cl.: A61B 19/00

(54) **Medizintechnische Instrumenten-Identifizierung**
Method and system for Identification of medical instruments
Méthode et système d'identification des instruments médicaux

(43) Veröffentlichungstag der Anmeldung: 06.08.2008
(73) Patentinhaber: BrainLAB AG, 85622 Feldkirchen (DE)
(72) Erfinder: Goldbach, Günter, 85457 Wörth/Wifling (DE)
(74) Vertreter: Rögner, Jürgen

(56) Entgegenhaltungen:
- WO-A-99/38449
- WO-A-2004/030560
- WO-A-2006/060631
- WO-A2-2004/001569
- DE-A1- 4 205 406
- DE-A1-102005 026 654
- US-A1- 2004 039 402
- US-A1- 2004 230 199
- US-A1- 2005 251 186
- US-A1- 2006 264 742

## Beschreibung

Die Erfindung betrifft die medizintechnische Instrumenten-Identifizierung. Im Umfeld einer medizintechnischen Navigation, bei der chirurgische Instrumente geortet, positionell verfolgt und zum Beispiel zur Bildunterstützung der Behandlung auf einem Bilddarstellungssystem (Monitor) zusammen mit Patientenstrukturen dargestellt werden, ist es oft notwendig und vorteilhaft, die Instrumente eindeutig identifizieren zu können. Dies gilt besonders in solchen Fällen, wo mit so genannten vorkalibrierten Instrumenten gearbeitet wird, also mit Instrumenten, deren äußere Form und Funktion schon vorab in einem Datenspeicher des medizintechnischen Navigationssystems hinterlegt sind. Hier ist eine eindeutige Identifizierung des Instruments notwendig. Allein aufgrund dieser Identifizierung hat das Navigationssystem dann ausreichend Informationen über das Instrument, um es korrekt navigieren zu können.

Es wurde beispielsweise in der WO 2004/001569 A2 schon vorgeschlagen, zur Identifizierung von solchen Instrumenten einen separaten Strichcodeleser zu verwenden. Nachteilig wirkt sich hier aus, dass die Instrumente immer erst an den Strichcodeleser herangeführt werden müssen, der einen relativ kleinen Sichtbereich aufweist. Ferner entstehen natürlich zusätzliche Kosten für die Anschaffung, das Handling und die Wartung des Strichcodelesers.

DE-A-102005026654 offenbart ein medizintechnisches System gemäß dem Oberbegriff der Ansprüche 1 und 10.

Es ist die Aufgabe der vorliegenden Erfindung, ein medizintechnisches Instrumenten-Identifizierungssystem sowie ein Verfahren zur Identifizierung medizinischer Instrumente bereitzustellen, welche im Bezug auf die Instrumentenerkennung optimiert sind. Insbesondere soll die Erfassung der Instrumente einfach und ohne zusätzliche oder neu zu installierende Sensoren ermöglicht werden.

Diese Aufgabe wird erfindungsgemäß durch ein medizintechnisches Instrumenten-Identifizierungssystem gemäß dem Anspruch 1 sowie ein Verfahren zur Identifizierung eines medizinischen Instruments gemäß dem Anspruch 10 gelöst. Die Unteransprüche definieren bevorzugte Ausführungsformen der Erfindung.

Ein erfindungsgemäßes System erkennt ein medizinisches Instrument, das im Rahmen einer medizintechnischen Navigation verwendet werden kann, und es weist einen oder mehrere optischen Sensor(en) und eine Datenverarbeitungsvorrichtung für die vom Sensor erfassten Identifikationsmerkmale des Instruments auf. Der optische Sensor und die Datenverarbeitungseinrichtung sind gemäß der vorliegenden Erfindung einem medizintechnischen Instrumententrackingsystem zugeordnet bzw. in dieses integriert, und die Datenverarbeitungseinrichtung umfasst eine videometrische Mustererkennung.

Bei navigationsgestützten Behandlungen wird ein Trackingsystem, wie es oben beschrieben wurde, ohnehin immer benützt, um die Instrumente zu orten und zu verfolgen. Die Erfindung integriert nunmehr eine weitere Funktion in das Trackingsystem, indem sie den optischen Sensor und die Datenverarbeitungsvorrichtung für die Instrumenten-Identifizierung ebenfalls diesem Instrumententrackingsystem zuordnet. Vorteilhafterweise wird dadurch schon vorhandene Hardware optimal genutzt, und weil der Sichtbereich solcher Trackingsysteme gewöhnlich relativ groß ist, müssen die Instrumente nicht separat einem Lesegerät vorgeführt werden, um sie in das Navigationsumfeld zu integrieren.

Bei einer Ausführungsform der Erfindung ist die Datenverarbeitungseinrichtung mit der Tracking-Datenverarbeitung des Trackingsystems verbunden oder integriert. Die Datenverarbeitung kann aber auch oder zusätzlich mit der Datenverarbeitung eines dem Trackingsystem zugeordneten, medizinischen Navigationssystems verbunden oder integriert sein.

Bei einer stark integrierten Ausführungsvariante kann der optische Sensor durch den Trackingsensor oder die Trackingsensoren des Trackingsystems gebildet werden. Ein Trackingsystem auf dem Gebiet der medizinischen Navigationstechnik weist oft ein stereoskopisches Kamerasystem auf, das Instrumente oder Apparaturen im Behandlungsumfeld der Position nach erfassen kann, und es kann gemäß dem obigen Vorschlag grundsätzlich auch die Identifizierungsfunktion übernehmen.

Ferner ist es ist im Rahmen der Erfindung möglich, als optischen Sensor einen zusätzlich zu den Trackingsensoren des Trackingsystems bereitgestellten, insbesondere am Trackingsystem angeordneten Sensor zu verwenden, speziell einen CMOS- oder CCD-Sensor.

Es besteht die Möglichkeit, auf dem Instrument ein oder mehrere Identifikationsmuster aufzubringen, insbesondere einen Strichcode, ein Textmuster oder eine Farbkennzeichnung, wobei auch andere optisch erfassbare Kennzeichnungen einsetzbar sind.

Das Identifikationsmuster kann auf einem auswechselbaren Teil, insbesondere auf einem austauschbaren Funktionsteil des Instruments aufgebracht werden, beispielsweise auf einer Steck-Bohrhülse, die auf einem (mit Referenzanordnung) navigierten Griff aufgesetzt werden kann. Mit Vorteil wird das Identifikationsmuster so aufgebracht, dass es von unterschiedlichen Blickwinkeln aus sichtbar ist und sich entsprechend über die Oberfläche des Instrumentes erstreckt.

Bei einer Gestaltungsvariante der Erfindung weist das Instrument oder ein Einzelteil davon oder die Gesamtheit seiner kombinierten Einzelteile eine aus einer Gruppe solcher Instrumente eindeutig identifizierbare Außenform auf. Ein Instrument kann also erfindungsgemäß durch ein oder mehrere aufgebrachte Muster oder durch seine Außenform (Kontur) oder aber auch durch beide Merkmale identifiziert werden.

Bei dem erfindungsgemäßen Verfahren zur Identifizierung eines medizinischen Instruments, das im Rahmen einer medizintechnischen Navigation verwendet werden kann, werden mit einem oder mehreren optischen Sensor(en), der bzw. die einem medizintechnischen Instrumenten-Trackingsystem zugeordnet ist bzw. sind, Identifikationsmerkmale des Instrumentes erfasst und einer Datenverarbeitungsvorrichtung zugeführt. Die Datenverarbeitungsvorrichtung ist ebenfalls dem medizintechnischen Instrumententrackingsystem zugeordnet. Die Daten über die Identifikationsmerkmale werden mittels einer videometrischen Mustererkennung verarbeitet und auf der Basis der verarbeiteten Daten wird das Instrument identifiziert. Das erfindungsgemäße Verfahren hat naturgemäß die Vorteile der erfindungsgemäßen Vorrichtung, und kann mit dem schon oben beschriebenen Vorrichtungselementen und -funktionen durchgeführt werden.

Bei einer Ausführungsform des Verfahrens wird ein mehrteiliges Instrument aus einer Gruppe zusammensetzbarer Instrumente, die ein gleiches Basisteil aufweisen, durch den Teil identifiziert, der die Kennzeichnung trägt oder eine einzigartige Außenform aufweist.

Eine weitere Ausführungsform der Erfindung ist dadurch gekennzeichnet, dass als optischer Sensor ein Trackingsensor oder Trackingsensoren des Trackingsystem und ein zusätzlich zu den Trackingsensoren des Trackingsystems bereitgestellter, insbesondere am Trackingsystem angebrachter, Sensor verwendet werden. Dabei kann die Datenverarbeitungsvorrichtung zur Identifizierung des Instruments entweder die Daten des Sensors oder der Sensoren auswerten, der bzw. die bei der momentanen Lage des Instruments die zuverlässigeren Daten liefert bzw. liefern, oder die Daten mehrerer Sensoren zusammenführen und gemeinsam auswerten.

Die Erfindung umfasst ferner ein Programm, das, wenn es auf einen Computer läuft oder in einem Computer geladen ist, den Computer veranlasst, ein Verfahren durchzuführen, wie es oben beschrieben wurde. Ein weiterer Aspekt der Erfindung besteht in einem Computerprogramm-Speichermedium, das ein solches Programm aufweist.

Ein wichtiges Element der vorliegenden Erfindung liegt darin, dass verfügbare Technologie für medizinische Anwendungen verwendet wird und unterschiedliche Technologien mit speziellen medizinischen Anwendungen kombiniert, bzw. integriert werden. Im vorliegenden Fall werden markerbasierte chirurgische Bildunterstützungssysteme bzw. medizintechnische Navigationssysteme, und speziell deren optische Trackingsysteme mit videometrischer Mustererkennungstechnologie kombiniert, um in einer neuen und verbesserten medizinischen Anwendung benützt zu werden, um so die Sicherheit und Effizienz der Systeme zu steigern. Ein Problem herkömmlicher optischer Trackingsysteme liegt nämlich darin, dass die Anzahl einsetzbarer, unterschiedlicher starrer Markergeometrien, die zur Instrumentenidentifizierung verwendet werden können, begrenzt ist. Ferner können bei der Identifizierung Probleme mit der Markererkennung, der Genauigkeit und Verlässlichkeit des Trackingsystems auftreten, sowie praktische Beschränkungen bei der Handhabung, was die Größe und das Gewicht der Instrumente bzw. Marker-Referenzanordnungen betrifft.

Durch die Erfindung wird die Funktionalität der Navigations-Trackingsysteme um bestimmte Arten der Mustererkennung in Kombination mit der Marker-Detektion erweitert. Dies bringt eine geeignete Ausleuchtung und die Erkennung charakteristischer Muster auf dem Instrument mit sich, sowie eine Einrichtung, welche die Informationen automatisch einer Werkzeug-Kombination zuordnen kann. Basierend auf der räumlichen Anordnung der starren Marker-Referenzanordnungen kann auch der Bereich, wo das Identifizierungsmuster zu erwarten ist, vorhergesagt werden, um die Verlässlichkeit des Systems zu verbessern.

Der Zweck der Verwendung der Identifikationsmerkmale der Instrumente, insbesondere der Identifikationsmuster, liegt speziell darin, mehrere Instrumente zu unterscheiden, welche dieselbe starre Referenzmarker-Geometrie oder -Anordnung nutzen, beispielsweise durch die Verwendung eines gemeinsamen Handgriffs, der mehrere Instrumenten-Funktionsteile tragen kann. Durch die Erkennung der ldentifikationsmerkmale am Instrument (Funktionsteil) kann das Trackingsystem spezielle geometrische Informationen für dieses besondere Instrument in einer Datenbank nachschlagen, und die Position der Instrumentenspitze bzw. des funktionellen Teils des Instruments kann errechnet werden, ohne dass eine separate Instrumentenkalibrierung erfolgen muss.

Die Vorteile des erfindungsgemäßen Systems liegen also unter anderem in der Verringerung der Anzahl erkennbarer Referenzanordnungen (Markeranordnungs-Geometrien), was sich insbesondere dann zeigt, wenn mehrere Versionen von Instrumenten mit derselben starren Referenzanordnung (Markergeometrie) verwendet werden. Ein gutes Beispiel ist eine Bohrhülse, wie sie speziell bei orthopädischen Anwendungen benutzt wird. Dieses Instrument besteht aus einem Handgriff, der eine Referenzgeometrie an seinen starren Basiskörper aufweist, sowie aus einer Hülse, die in mehreren Variationen verfügbar ist, und zwar abhängig vom Bohrerdurchmesser und der Schaftlänge. Für den Chirurgen ist es nunmehr wichtig, sicherzustellen, dass das Trackingsystem bzw. das Navigationssystem weiß, welche Instrumentenkombination zu einem bestimmten Zeitpunkt tatsächlich verwendet wird. Ansonsten wäre es nicht möglich, vorkalibrierte Instrumente zu verwenden, und die Spitze jedes Instrumentes müsste relativ zum getrackten starren Basiskörper (zur Referenzanordnung) kalibriert werden. Gemäß dem Stand der Technik wurden hier für den Benutzer oftmals Listen auf dem Navigationsmonitor bereitgestellt, wo er das tatsächliche Instrument auswählen konnte. Nachteiligerweise bedingt dies aber eine weitere Interaktion mit dem System und birgt Fehlerquellen.

Die vorliegende, erfindungsgemäße Instrumenten-Identifizierung verringert in dieser Hinsicht die Interaktion mit dem Navigationssystem und vereinfacht somit den Arbeitsablauf. Sie verringert auch das Risiko der manuellen Auswahl eines falschen Instruments, was schwere Probleme mit sich bringen könnte. Sie verkleinert den Aufwand, bekannte und oft verwendete Instrumente und chirurgische Werkzeuge zu modifizieren; es wird kein zusätzliches Gewicht angebracht, und es besteht kein Bedarf an zusätzlichen Teilen oder Markern. Die Erfindung hat keinen negativen Einfluss auf die Sicherheit und Zuverlässigkeit des Instruments selbst, und sie bringt keine wesentlichen Kostenerhöhungen mit sich, da nur geringe Modifikationen existierender Systeme durchgeführt werden müssen. Ferner können existierende Instrumente oder Werkzeuge mit einem "Upgrade" erfindungsgemäß angepasst werden, und zwar zu relativ geringen Kosten, weil oft nur ein Muster auf dem Instrument angebracht werden muss. Es können Techniken zur Ausstattung des Instruments mit Identifizierungsmerkmalen verwendet werden, die weder die Sterilität des Instruments negativ beeinflussen, noch dessen Desinfektion oder Reinigung erschweren, beispielsweise eine Lasergravierung auf Edelstahlinstrumenten.

Die Erfindung wird im Weiteren anhand der beiliegenden Zeichnungen und verschiedener Ausführungsformen näher beschrieben. Sie kann alle hierin aufgeführten Merkmale einzeln sowie in jedweder sinnvollen Kombination umfassen. In den Zeichnungen zeigen:
- Figur 1: ein erfindungsgemäßes medizinisches Instrumenten-Identifizierungssystem mit seinen Hauptkomponenten in schematischer Darstellung; und
- Figuren 2 und 3: Beispiele für medizinische Instrumente, die mit einem erfindungsgemäßen ldentifikationsmuster versehen sind.

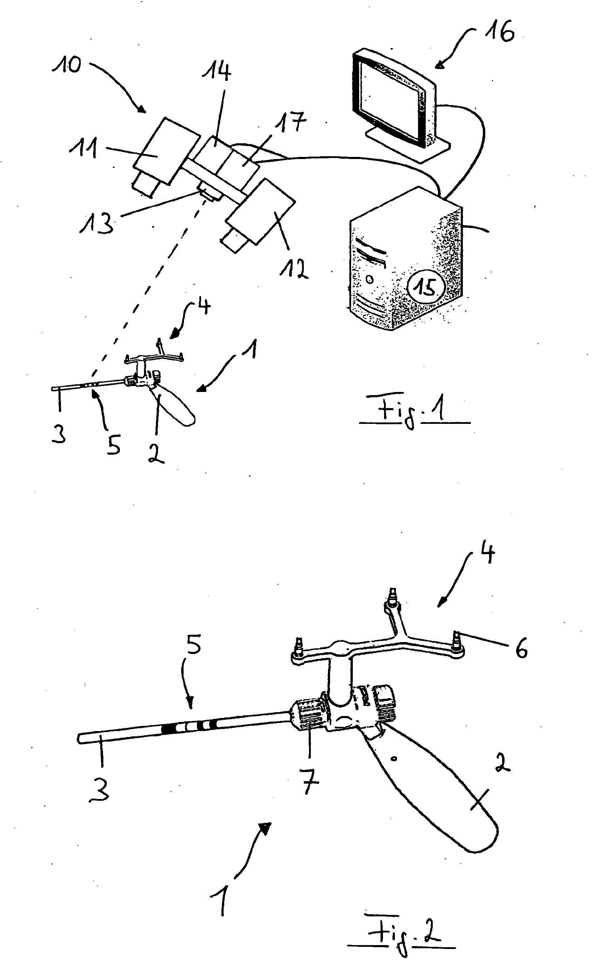

In der Figur 1 ist ein chirurgisches Instrument mit dem Bezugszeichen 1 angedeutet. Es hat einen Handgriff 2 sowie eine Navigations-Referenzanordnung 4, auf der beispielsweise drei Reflektionsmarker-Kugeln aufgebracht werden können. Der Griff und die Referenzanordnung 4 sind starr miteinander verbunden und bilden zusammen das Basisteil des Instruments.

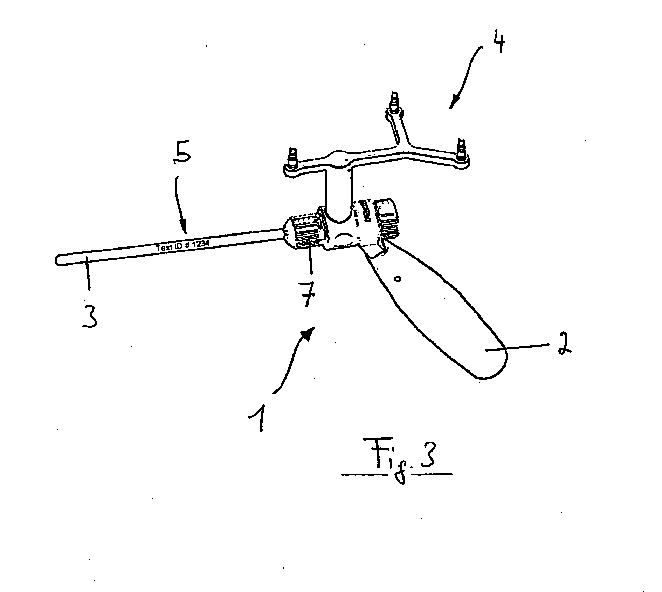

Zusätzlich weist das Instrument noch ein auswechselbares Funktionsteil auf, nämlich im vorliegenden Fall, wobei es sich um eine chirurgische Bohrerführung handelt, eine Bohrhülse 3. Die Bohrhülse 3 kann auf das Basisteil 2, 4 aufgesteckt werden, und sie weist hierzu den Adapter 7 auf (siehe Figuren 2 und 3). Die obigen Bezugszeichen gelten nicht nur für die Figur 1 sondern auch für die beiden unterschiedlichen Instrumente, die in den Figuren 2 und 3 dargestellt sind.

Das Instrument 1 wird in einem Navigationsumfeld geortet und positionell verfolgt, und zwar durch das Trackingsystem 10. Das Trackingsystem 10 ist ein stereoskopisches Trackingsystem mit zwei Kameras 11, 12, welche Marker (nicht dargestellt) positionell erfassen, die auf der Referenzanordnung 4 des Instruments 1 angeordnet sind. Durch die stereoskopische Erfassung kann die Lage des Instrumentes im Raum koordinatenmäßig und dreidimensional erfasst werden. Wie bei üblichen Navigationssystemen verarbeitet das Trackingsystem 10 in einer Datenverarbeitungseinheit 17 die Positionsdaten und gibt sie an ein Navigationssystem weiter, das hier schematisch durch einen Computer 15 und einen Monitor 16 dargestellt ist.

Zusätzlich und gemäß einer Ausführungsform der Erfindung wird eine an dem Trackingsystem 10 angebrachte Videokamera 13 verwendet, um ein Abbild eines Identifikationsmusters 5 aufzunehmen, das an der Hülse 3 des Instruments 1 aufgebracht ist. Das Muster 5 hat im vorliegenden Fall die Form einer Art Strichcode und geht rings um die Bohrhülse 3 herum, damit es von allen Seiten des Instruments her sichtbar ist. Die Erfindung ist nicht auf eine solche Strichcode-Anordnung zur Identifizierung beschränkt. Es können auch Textmarkierungen verwendet werden, und eine solche Ausführungsform ist beispielsweise in der Figur 3 zu sehen. Auch der Text kann mehrfach und rund um den Instrumentenschaft (Bohrhülse 3) herum angeordnet werden. Wenn unterschiedliche Bohrhülsen verwendet werden, werden diese unterschiedliche Identifizierungsmerkmale haben, und durch die vorher beschriebene Erfassung mit Hilfe der Kamera (CMOS- oder CCD-Sensor) 13 wird dem Navigationssystem 15 mitgeteilt, welche Bohrhülse gerade im Einsatz ist. Das Navigationssystem hat eine Liste gespeichert, welche verschiedene Bohrhülsen umfasst, und es kennt damit die Länge und Anordnung jeder einzelnen Bohrhülse am Instrument 1 sowie deren Spitzen-Position und die räumliche Anordnung (über die Referenzanordnung 4). Im in Figur 1 dargestellten Beispiel ist am Trackingsystem zusätzlich schematisch eine Datenverarbeitungseinrichtung 14 angedeutet worden, welche dafür zuständig ist, die Identifikationsmerkmale zu verarbeiten und an das Navigationssystem 15 weiterzugeben. Es ist aber grundsätzlich auch möglich, mit dem Trackingsystem lediglich die ldentifikationsmerkmale zu erfassen und die weitere Verarbeitung gänzlich im Navigationssystem 15 durchzuführen. Natürlich können die Datenverarbeitungseinrichtungen 14 (Identifizierung) und 17 (Navigation) auch zu einer einzigen Datenverarbeitungseinheit mit beiden Funktionen vereint werden.

Die Mustererkennung kann im Bereich des sichtbaren Lichts durchgeführt werden, oder bei unsichtbaren Wellenlängen im Infrarot- oder UV-Bereich. Es ist grundsätzlich möglich, die separate Bilderfassungseinrichtung 13 wegzulassen und auch die Erfassung des Identifikationsmusters 5 von den Tracking-Kameras 11, 12 durchführen zu lassen. Hierzu bestünde die Möglichkeit, die Ausleuchtung oder Verstärkung sowie die Verschluss-Einstellungen der Trackingsystem-Kameras so anzupassen, dass sie sowohl ihre Trackingfunktion als auch die Identifizierungsfunktion erfüllen können.

## Patentansprüche

1. Medizintechnisches Instrumenten-Identifizierungssystem, welches ein medizinisches Instrument (1) erkennt, das im Rahmen einer medizintechnischen Navigation verwendet werden kann, mit einem oder mehreren optischen Sensor(en) (11, 12, 13) und einer Datenverarbeitungsvorrichtung (14, 15) für die vom optischen Sensor (11, 12, 13) erfassten Identifikationsmerkmale des Instruments (1), wobei unterschiedliche Arten von Identifikationsmerkmalen erfasst werden und der optische Sensor (11, 12, 13) und die Datenverarbeitungsvorrichtung (14, 15) einem medizintechnischen Instrumententrackingsystem (10) zugeordnet sind, und dass die Datenverarbeitungseinrichtung (14) zusätzlich zu einer ersten Art von Instrumentenidentifizierung, nämlich eine Referenzmarker-Geometrie-Detektion, eine weitere, von der ersten Art unterschiedliche Art von Instrumentenidentifizierung, nämlich eine videometrische Mustererkennung umfasst, **dadurch gekennzeichnet, dass** das Instrumententrackingsystem (10) zur Kalibrierung des Instruments (1) auf Basis der Erkennung der Identifizierungsmerkmale geometrische Informationen für das Instrument (1) in einer Datenbank nachschlägt und dass der Bereich, wo das Identifizierungsmuster zu erwarten ist, vorhergesagt wird.

2. Medizintechnisches Instrumenten-Identifizierungssystem nach Anspruch 1, **dadurch gekennzeichnet, dass** die Datenverarbeitungseinrichtung (14) mit der Tracking-Datenverarbeitung (17) des Trackingsystems verbunden oder integriert ist.

3. Medizintechnisches Instrumenten-Identifizierungssystem nach Anspruch 1 oder 2, **dadurch gekennzeichnet, dass** die Datenverarbeitungseinrichtung (14) mit der Datenverarbeitung eines dem Trackingsystem zugeordneten, medizintechnischen Navigationssystems verbunden oder integriert ist.

4. Medizintechnisches Instrumenten-Identifizierungssystem nach einem der Ansprüche 1 bis 3, **dadurch gekennzeichnet, dass** der optische Sensor durch den Trackingsensor oder die Trackingsensoren des Trackingsystem gebildet wird.

5. Medizintechnisches Instrumenten-Identifizierungssystem nach einem der Ansprüche 1 bis 4, **dadurch gekennzeichnet, dass** der optische Sensor einen zusätzlich zu den Trackingsensoren (11, 12) des Trackingsystems (10) bereitgestellten, insbesondere am Trackingsystem angeordneteh Sensor (13) umfasst, speziell einen CMOS- oder CCD-Sensor.

6. Medizintechnisches Instrumenten-Identifizierungssystem nach einem der Ansprüche 1 bis 5, **dadurch gekennzeichnet, dass** auf dem Instrument (1) ein oder mehrere Identifikationsmuster (5) aufgebracht ist bzw. sind, insbesondere ein Strichcode, ein Textmuster oder eine Farbkennzeichnung.

7. Medizintechnisches Instrumenten-Identifizierungssystem nach Anspruch 6, **dadurch gekennzeichnet, dass** das Identifikationsmuster (5) auf einem auswechselbaren Teil, insbesondere auf einem austauschbaren Funktionsteil (3) des Instruments (1) aufgebracht ist.

8. Medizintechnisches Instrumenten-Identifizierungssystem nach Anspruch 6 oder 7, **dadurch gekennzeichnet, dass** das Identifikationsmuster (5) sich von unterschiedlichen Blickwinkeln aus sichtbar über die Oberfläche des Instruments erstreckt.

9. Medizintechnisches Instrumenten-Identifizierungssystem nach einem der Ansprüche 1 bis 8, **dadurch gekennzeichnet, dass** das Instrument (1) oder ein Einzelteil davon oder die Gesamtheit seiner kombinierten Einzelteile eine aus einer Gruppe solcher Instrumente eindeutig identfizierbare Außenform aufweist.

10. Verfahren zur Identifizierung eines medizinischen Instruments, das im Rahmen einer medizintechnischen Navigation verwendet werden kann, bei dem mit einem oder mehreren optischen Sensor(en) (11, 12, 13), der einem medizintechnischen Instrumententrackingsystem (10) zugeordnet ist, unterschiedliche Arten von Identifikationsmerkmalen des Instruments (1) erfasst und einer Datenverarbeitungsvorrichtung (14, 15) zugeführt werden, die ebenfalls dem medizintechnischen Instrumententrackingsystem (10) zugeordnet ist, und wobei die Daten zusätzlich zu einer ersten Art von Instrumentenidentifizierung über Identifikationsmerkmale, nämlich einer Referenzmarker-Geometrie-Detektion mittels einer weiteren, von der ersten Art unterschiedlichen Art von Instrumentenidentifizierung, nämlich einer videometrischen Mustererkennung verarbeitet werden und auf der Basis der verarbeiteten Daten das Instrument identifiziert wird, **dadurch gekennzeichnet, dass** zur Kalibrierung des Instruments (1) auf Basis der Erkennung der Identifikationsmerkmale geometrische Informationen für das Instrument (1) durch das Instrumententrackingsystem (10) in einer Datenbank nachgeschlagen werden und dass der Bereich, wo das Identifizierungsmuster zu erwarten ist, vorhergesagt wird.

11. Verfahren nach Anspruch 10, bei dem als optischer Sensor ein zusätzlich zu den Trackingsensoren (11, 12) des Trackingsystems (10) bereitgestellter, insbesondere am Trackingsystem angebrachter, Sensor (13) verwendet wird, speziell ein CMOS oder CCD-Sensor.

12. Verfahren nach Anspruch 10 oder 11, bei dem als optischer Sensor ein Trackingsensor oder Trackingsensoren (11, 12) des Trackingsystems verwendet wird bzw. werden.

13. Verfahren nach Anspruch 10, bei dem als optischer Sensor ein Trackingsensor oder Trackingsensoren (11, 12) des Trackingsystems und ein zusätzlich zu den Trackingsensoren (11, 12) des Trackingsystems (10) bereitgestellter, insbesondere am Trackingsystem angebrachter, Sensor verwendet werden.

14. Verfahren nach Anspruch 13, bei dem die Datenverarbeitungsvorrichtung (14, 15) zur Identifizierung des Instruments (1)
- die Daten des Sensors oder der Sensoren auswertet, der bzw. die bei der momentanen Lage des Instruments die zuverlässigeren Daten liefert bzw. liefern; oder
- die Daten mehrerer Sensoren zusammenführt und gemeinsam auswertet.

15. Verfahren nach einem der Ansprüche 10 bis 14, bei dem das Instrument (1) durch ein oder mehrere Identifikationsmuster (5) identifiziert wird, insbesondere durch einen Strichcode, ein Textmuster oder eine Farbkennzeichnung.

16. Verfahren nach einem der Ansprüche 10 bis 15, bei dem das Instrument (1) durch die Außenform eines seiner Einzelteile oder die Außenform der Gesamtheit seiner kombinierten Einzelteile aus einer Gruppe solcher Instrumente eindeutig identifiziert wird.

17. Verfahren nach einem der Ansprüche 10 bis 16, bei dem ein mehrteiliges Instrument (1) aus einer Gruppe zusammensetzbarer Instrumente, die ein gleiches Basisteil aufweisen, durch den Teil identifiziert wird, der die Kennzeichnung trägt oder eine einzigartige Außenform aufweist.

18. Programm, das, wenn es auf einem Computer eines medizintechnischen Navigationssystems läuft oder in einem Computer eines medizintechnischen Navigationssystems geladen ist, den Computer veranlasst, ein Verfahren gemäß einem der Ansprüche 10 bis 17 durchzuführen.

19. Computerprogramm-Speichermedium, das ein Programm nach Anspruch 18 aufweist.

## Claims

1. A medical instrument identification system which recognises a medical instrument (1) which can be used within the framework of medical navigation, comprising one or more optical sensor(s) (11, 12, 13) and a data processing device (14, 15) for the identification features of the instrument (1) which are detected by the optical sensor (11, 12, 13), wherein different types of identification features are detected and the optical sensor (11, 12, 13) and the data processing device (14, 15) are assigned to a medical instrument tracking system (10), and wherein the data processing means (14) comprises - in addition to a first type of instrument identification, namely reference marker geometry detection - another type of instrument identification which is different from the first type, namely videometric pattern recognition, **characterised in that** the instrument tracking system (10) looks up geometric information for the instrument (1) in a database in order to calibrate the instrument (1) on the basis of recognising the identification features, and **in that** the area in which the identification pattern may be expected is predicted.

2. The medical instrument identification system according to claim 1, **characterised in that** the data processing means (14) is connected to or integrated into the tracking data processing (17) of the tracking system.

3. The medical instrument identification system according to claim 1 or 2, **characterised in that** the data processing means (14) is connected to or integrated into the data processing of a medical navigation system assigned to the tracking system.

4. The medical instrument identification system according to any one of claims 1 to 3, **characterised in that** the optical sensor is formed by the tracking sensor or tracking sensors of the tracking system.

5. The medical instrument identification system according to any one of claims 1 to 4, **characterised in that** the optical sensor comprises a sensor (13), specifically a CMOS or CCD sensor, which is provided in addition to the tracking sensors (11, 12) of the tracking system (10) and is in particular arranged on the tracking system.

6. The medical instrument identification system according to any one of claims 1 to 5, **characterised in that** one or more identification patterns (5), in particular a barcode, a text pattern or a colour code, is/are attached to the instrument (1).

7. The medical instrument identification system according to claim 6, **characterised in that** the identification pattern (5) is attached to a replaceable part, in particular an exchangeable functional part (3), of the instrument (1).

8. The medical instrument identification system according to claim 6 or 7, **characterised in that** the identification pattern (5) extends over the surface of the instrument, visible from different angles of view.

9. The medical instrument identification system according to any one of claims 1 to 8, **characterised in that** the instrument (1) or an individual part of it or the entirety of its combined individual parts exhibits an external shape which can be unambiguously identified from a group of such instruments.

10. A method for identifying a medical instrument which can be used within the framework of medical navigation, wherein different types of identification features of the instrument (1) are detected using one or more optical sensor(s) (11, 12, 13) assigned to a medical instrument tracking system (10), and are fed to a data processing device (14, 15) which is also assigned to the medical instrument tracking system (10), and wherein the data concerning identification features are processed - in addition to a first type of instrument identification, namely reference marker geometry detection - by means of another type of instrument identification which is different from the first type, namely videometric pattern recognition, and the instrument is identified on the basis of the processed data, **characterised in that** geometric information for the instrument (1) is looked up in a database by the instrument tracking system (10) in order to calibrate the instrument (1) on the basis of recognising the identification features, and **in that** the area in which the identification pattern may be expected is predicted.

11. The method according to claim 10, wherein a sensor (13) which is provided in addition to the tracking sensors (11, 12) of the tracking system (10) and is in particular attached to the tracking system, specifically a CMOS or CCD sensor, is used as the optical sensor.

12. The method according to claim 10 or 11, wherein a tracking sensor or tracking sensors (11, 12) of the tracking system is/are used as the optical sensor.

13. The method according to claim 10, wherein a tracking sensor or tracking sensors (11, 12) of the tracking system and a sensor which is provided in addition to the tracking sensors (11, 12) of the tracking system (10) and is in particular attached to the tracking system are used as the optical sensor.

14. The method according to claim 13, wherein the data processing device (14, 15) for identifying the instrument (1):
- evaluates the data of the sensor or sensors providing the more reliable data, given the current location of the instrument; or
- combines the data of a number of sensors and evaluates them together.

15. The method according to any one of claims 10 to 14, wherein the instrument (1) is identified by one or more identification patterns (5), in particular a barcode, a text pattern or a colour code.

16. The method according to any one of claims 10 to 15, wherein the instrument (1) is unambiguously identified from a group of such instruments by the external shape of one of its individual parts or the external shape of the entirety of its combined individual parts.

17. The method according to any one of claims 10 to 16, wherein a multiple-part instrument (1) consisting of a group of instruments which can be assembled and which comprise the same base part is identified by the part which bears the code or which exhibits a unique external shape.

18. A program which, when it is running on a computer of a medical navigation system or is loaded on a computer of a medical navigation system, causes the computer to perform a method in accordance with any one of claims 10 to 17.

19. A computer program storage medium comprising a program according to claim 18.

## Revendications

1. Système d'identification d'instrument médical, lequel reconnaît un instrument médical (1) qui peut être utilisé dans le cadre d'une navigation médicale, comportant un ou plusieurs capteurs optiques (11, 12, 13) et un dispositif de traitement de données (14, 15) pour les caractéristiques d'identification de l'instrument (1) détectées par le capteur optique (11, 12, 13), dans lequel différents types de caractéristiques d'identification sont détectés et le capteur optique (11, 12, 13) et le dispositif de traitement de données (14, 15) sont couplés à un système de repérage d'instrument médical (10), et en ce que le dispositif de traitement de données (14) inclut, en plus d'un premier type d'identification d'instrument, à savoir une détection de géométrie de marqueur de référence, un autre type d'identification d'instrument différent du premier type, à savoir une reconnaissance de forme vidéométrique, **caractérisé en ce que** le système de repérage d'instrument (10) recherche des informations géométriques concernant l'instrument (1) dans une base de données afin de calibrer l'instrument (1) sur la base de la reconnaissance des caractéristiques d'identification, et **en ce que** la zone où la forme d'identification est attendue, est prédite.

2. Système d'identification d'instrument médical selon la revendication 1, **caractérisé en ce que** le dispositif de traitement de données (14) est relié à une unité de traitement de données de repérage (17) du système de repérage ou est intégré dans celle-ci.

3. Système d'identification d'instrument médical selon la revendication 1 ou 2, **caractérisé en ce que** le dispositif de traitement de données (14) est relié à l'unité de traitement de données d'un système de navigation médical couplé au système de repérage, ou est intégré dans celle-ci.

4. Système d'identification d'instrument médical selon l'une quelconque des revendications 1 à 3, **caractérisé en ce que** le capteur optique est formé par le capteur de repérage ou les capteurs de repérage du système de repérage.

5. Système d'identification d'instrument médical selon l'une quelconque des revendications 1 à 4, **caractérisé en ce que** le capteur optique inclut un capteur (13) disposé en particulier sur le système de repérage, fourni en plus des capteurs de repérage (11, 12) du système de repérage (10), notamment un capteur CMOS ou CCD.

6. Système d'identification d'instrument médical selon l'une quelconque des revendications 1 à 5, **caractérisé en ce qu'**une ou plusieurs formes d'identification (5) sont appliquées sur l'instrument (1), en particulier un code à barres, un modèle de texte ou un code de couleurs.

7. Système d'identification d'instrument médical selon la revendication 6, **caractérisé en ce que** la forme d'identification (5) est appliquée sur une partie remplaçable, en particulier sur une partie fonctionnelle échangeable (3) de l'instrument (1).

8. Système d'identification d'instrument médical selon la revendication 6 ou 7, **caractérisé en ce que** la forme d'identification (5) s'étend à partir de différents angles de vue visibles sur la surface de l'instrument.

9. Système d'identification d'instrument médical selon l'une quelconque des revendications 1 à 8, **caractérisé en ce que** l'instrument (1) ou une partie individuelle de celui-ci ou la totalité de ses parties individuelles combinées présente une forme extérieure pouvant être clairement identifiée parmi un groupe de tels instruments.

10. Procédé d'identification d'un instrument médical, lequel peut être utilisé dans le cadre d'une navigation médicale, dans lequel au moyen d'un ou plusieurs capteurs optiques (11, 12, 13) couplés à un système de repérage d'instrument médical (10), différents types de caractéristiques d'identification de l'instrument (1) sont détectés et sont transmis à un dispositif de traitement de données (14, 15), lequel dispositif de traitement de données est également couplé au système de repérage d'instrument médical (10), et dans lequel les données sont traitées, en plus d'un premier type d'identification d'instrument concernant des caractéristiques d'identification, à savoir une détection de géométrie de marqueur de référence, au moyen d'un autre type d'identification d'instrument différent du premier type, à savoir une reconnaissance de forme vidéométrique, et l'instrument est identifié sur la base des données traitées, **caractérisé en ce que** le système de repérage d'instrument (10) recherche des informations géométriques concernant l'instrument (1) dans une base de données afin de calibrer l'instrument (1) sur la base de la reconnaissance des caractéristiques d'identification, et **en ce que** la zone où la forme d'identification est attendue, est prédite.

11. Procédé selon la revendication 10, dans lequel un capteur (13), en particulier fixé sur le système de repérage, fourni en plus du capteur de repérage (11, 12) du système de repérage (10), est utilisé comme capteur optique, en particulier un capteur CMOS ou CCD.

12. Procédé selon la revendication 10 ou 11, dans lequel un capteur de repérage ou des capteurs de repérage (11, 12) du système de repérage sont utilisés comme capteurs optiques.

13. Procédé selon la revendication 10, dans lequel un capteur de repérage ou des capteurs de repérage (11, 12) du système de repérage et un capteur, en particulier fixé sur le système de repérage, fourni en plus du capteur de repérage (11, 12) du système de repérage (10), sont utilisés comme capteurs optiques.

14. Procédé selon la revendication 13, dans lequel le dispositif de traitement de données (14, 15) pour identifier l'instrument (1)
- évalue les données du capteur ou des capteurs qui fournit ou fournissent les données fiables par la position instantanée de l'instrument ; ou
- collecte les données de plusieurs capteurs et les évalue collectivement.

15. Procédé selon l'une quelconque des revendications 10 à 14, dans lequel l'instrument (1) est identifié par une ou plusieurs formes d'identification (5), en particulier par un code à barres, un modèle de texte ou un code de couleurs.

16. Procédé selon l'une quelconque des revendications 10 à 15, dans lequel l'instrument (1) est clairement identifié par la forme extérieure de l'une de ses parties individuelles ou par la forme extérieure de la totalité de ses parties individuelles combinées parmi un groupe de tels instruments.

17. Procédé selon l'une quelconque des revendications 10 à 16, dans lequel un instrument en plusieurs parties (1) parmi un groupe d'instruments pouvant être regroupés et comportant une partie de base identique, est identifié par la partie qui porte le code ou qui présente une forme extérieure unique.

18. Programme qui, lorsqu'il s'exécute sur un ordinateur d'un système de navigation médical ou qu'il est chargé dans un ordinateur d'un système de navigation médical, amène l'ordinateur à mettre en oeuvre un procédé selon l'une quelconque des revendications 10 à 17.

19. Support de mémorisation de programme d'ordinateur comportant un programme selon la revendication 18.
